(19) Europäisches Patentamt
European Patent Office
Office européen des brevets

(11) Publication number: **0 208 374 B1**

(12) **EUROPEAN PATENT SPECIFICATION**

(45) Date of publication of patent specification: **02.05.91**

(21) Application number: **86201147.5**

(22) Date of filing: **28.09.83**

(51) Int. Cl.[5]: **C07D 231/56**, A01N 43/56

(60) Publication number of the earlier application in accordance with Art.76 EPC: **0 105 721**

(54) **2,4-Dihalo-5-nitrophenyl-4,5,6,7-tetrahydro-2H-indazoles, and their production and use.**

(30) Priority: **28.09.82 JP 170427/82**
**06.11.82 JP 194893/82**
**16.03.83 JP 45027/83**

(43) Date of publication of application:
**14.01.87 Bulletin 87/03**

(45) Publication of the grant of the patent:
**02.05.91 Bulletin 91/18**

(84) Designated Contracting States:
**CH DE FR IT LI**

(56) References cited:
**FR-A- 2 327 990**
**FR-A- 2 338 263**
**US-A- 4 179 276**

(73) Proprietor: **SUMITOMO CHEMICAL COMPANY, LIMITED**
**Kitahama 4-chome 5-33**
**Chuo-ku Osaka 541(JP)**

(72) Inventor: **Nagano, Eiki**
**4-1-401, Ryodo-cho**
**Nishinomiya Hyogo(JP)**

Inventor: **Takemoto, Ichiki**
**14-7, Mefu 2-chome**
**Takarazuka Hyogo(JP)**
Inventor: **Fukushima, Masayuki**
**14-7, Mefu 2-chome**
**Takarazuka Hyogo(JP)**
Inventor: **Yoshida, Ryo**
**5-19, Azajizoyama Higashiueno**
**Kawanishi Hyogo(JP)**
Inventor: **Matsumoto, Hiroshi c/o Sumitomo Chemical Shataku**
**No. 15-10-305, Kusunoki-cho**
**Ashiya-shi Hyogo-ken(JP)**

(74) Representative: **Allard, Susan Joyce**
**BOULT, WADE & TENNANT, 27 Furnival Street**
**London EC4A 1PQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to 2,4-dihalo-5-nitrophenyl-4,5,6,7-tetrahydro-2H-indazoles, and their production and use.

The said indazoles are representable by the formula:

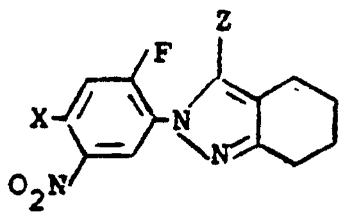

(XI)

wherein X is a chlorine atom or a bromine atom, and Z is a chlorine atom or a methyl group.
The compounds of the present invention are intermediates in the production of 2-substituted phenyl-4,5,6,7-tetrahydro-2H-indazoles which possess herbicidal activity and which have the general formula

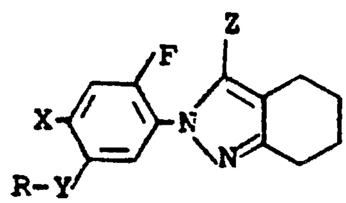

(I)

wherein X is a chlorine atom or a bromine atom, Y is an oxygen atom or an imino group, Z is a chlorine atom or a methyl group and R is an alkyl group of more than one but not more than four carbon atoms, a $C_3$-$C_4$ alkenyl group, a $C_3$-$C_4$ alkynyl group, a $C_1$-$C_6$ alkoxycarbonylmethyl group, a $C_3$-$C_6$ cycloalkoxycarbonylmethyl group or a $C_1$-$C_4$haloalkoxycarbonylmethyl group.

These compounds have a strong herbicidal activity against a wide variety of weeds including broad-leaved weeds and Graminaceous weeds in agricultural ploughed fields as well as weeds in paddy fields at small doses and do not produce any significant phytotoxicity to various agricultural crops (i.e. corn, wheat, soybean, cotton and rice plants). These compounds and their preparation and use are described in our co-pending European Patent No. 105 721.

The indazole (I) may be obtained by reacting a 2,4-dihalo-5-substituted phenyl-4,5,6,7-tetrahydro-2H-indazole of the formula:

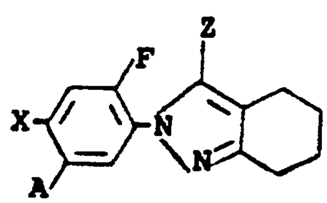

(II)

wherein X and Z are each as defined above and A is an amino group or a hydroxyl group with a halogenated compound of the formula:

R-B (III)

wherein R is as defined above and B is a halogen atom.

The 2,4-dihalo-5-substituted phenyl-4,5,6,7-tetrahydro-2H-indazole (II) wherein A is an amino group is obtainable by reacting a 2,4-dihalo-5-nitrophenylhydrazine of the formula:

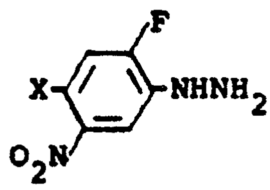

(IX)

wherein X is as defined above with a 2-alkoxycarbonylcyclohexanone of the formula:

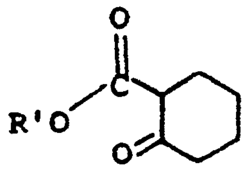

(V)

wherein R' is a $C_1$-$C_4$ alkyl group to give a 2-(2,4-dihalo-5-nitrophenyl)hexahydroindazol-3-one of the formula:

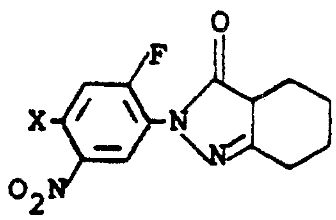

(X)

wherein X is as defined above, followed by reacting the latter with a chlorinating agent to give the compound of the formula:

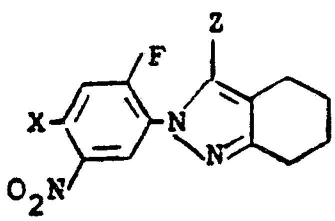

(XI)

wherein X is as defined above and Z is a chlorine atom.

The first reaction may be carried out in a solvent at a temperature of 80 to 200° for a period of 0.5 to 20 hours. The 2-alkoxycarbonylcyclohexanone (V) is usually employed in an amount of 1 to 1.2 equivalents to the substituted phenylhydrazine (IX). Examples of the solvent are toluene, xylene, acetic acid, etc.

The second reaction is normally carried out in a solvent at a temperature of 80 to 200° C for a period of 5 to 20 hours. The chlorinating agent may be employed in an excess amount to the intermediary product (X). Examples of the chlorinating agent are phosphorus oxychloride, thionyl chloride, phosgene, oxalic dichloride, trichloromethyl chloroformate, etc. Examples of the solvent are toluene, xylene, chloroform, etc.

The compound of formula (XI) is then subjected to reduction with an acid (e.g. hydrochloric acid or acetic acid) and iron powder.

Alternatively, the 2,4-dihalo-5-nitrophenyl-hydrazine (IX) may be reacted with 2-acetyl-cyclohexanone as in the manner as described above to give a 2,4-dihalo-5-nitrophenyl-4,5,6,7-tetrahydro-2H-indazole of the formula:

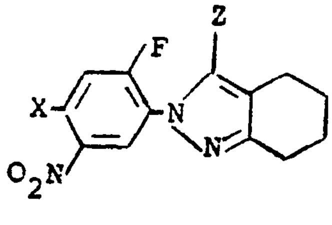

(XI)

wherein X is as defined above and Z is as defined above and Z is a methyl group, followed by subjecting the latter to reduction by a per se conventional procedure such as reduction with an acid (e.g. hydrochloric acid, acetic acid) and iron powder.

The intermediary 2,4-diahlo-5-nitro-phenylhydrazine (IX), may be produced according to the following scheme:

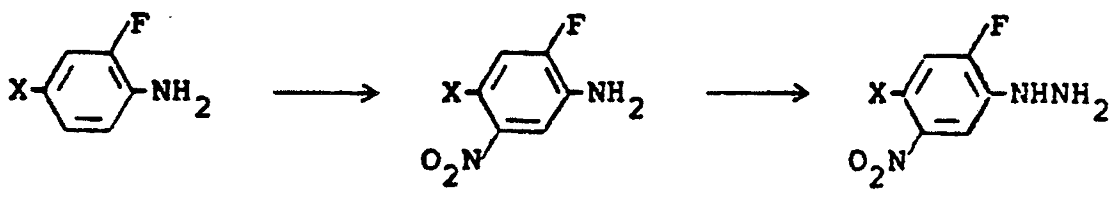

(XII) (XIII) (IX)

wherein X is each as defined above.

The above conversion is known and described in the art.

The present invention is further described with reference to the following Examples.

Example 1

Production of the 2,4-dihalo-5-nitrophenyl-4,5,6,7-tetrahydro-2H-indazole (XI):-

4-Chloro-2-fluoro-5-nitrophenylhydrazine (4 g), 2-acetylcyclohexanone (2.8 g) and a catalytic amount of acetic acid were admixed with xylene (10 ml). The resultant mixture was heated under reflux for 5 hours while removing water. After cooling, the mixture was concentrated and the residue was purified by silica gel column chromatography to give 2.3 g of 2-(4-chloro-2-fluoro-5-nitrophenyl)-3-methyl -4,5,6,7-tetrahydro-2H-indazole. M.P., 143 - 144° C.

Example 2

Production of the 2,4-dihalo-5-nitrophenyl-4,5,6,7-tetrahydro-2H-indazole (XI):-

A solution of 4-chloro-2-fluoro-5-nitrophenyl-hydrazine (4.6 g) and 2-ethoxycarbonylcyclohexanone (3.9 g) in acetic acid (20 ml) was heated under reflux for 4 hours. After cooling, the precipitated crystals were collected by filtration and washed with ether to give 4.2 g of 2-(4-chloro-2-fluoro-5-nitrophenyl)-2,3a,4,5,6,7-hexahydroisoindazol-3-one.

The thus obtained product was added to a IM solution of phosgene in toluene (300 ml) and the resultant mixture was heated under reflux for 3 hours. After cooling, the mixture was concentrated and the residue was purified by silica gel column chromatography to give 0.8 g of 3-chloro-2-(4-chloro-2-fluoro-5-nitrophenyl)-4,5,6,7-tetrahydro-2H-indazole. M.P., 120 - 122° C.

Example 3

Production of the nitroaniline (XIII):-

4-Chloro-2-fluoroaniline (23 g) was dissolved in conc, sulfuric acid (120 ml), and the resultant mixture was cooled to -20° C, followed by dropwise addition of fuming nitric acid (15 g). The reaction mixture was stirred at -20 to -15° C for 1.5 hours, poured into ice-water and then extracted with ether. The ether extract was washed with water and a saturated sodium bicarbonate solution, dried and concentrated. The residue was crystallized from a mixture of toluene and hexane (2 : 1) to obtain 20 g of 4-chloro-2-fluoro-5-nitroaniline. M.P., 83 - 84.5° C.

Example 4

In the same manner as in Example 3 but using 4-bromo-2-fluoroaniline, there was obtained 4-bromo-2-fluoro-5-nitroaniline. M.P., 90 - 92° C.

## Claims

**1.** A 2,4-dihalo-5-nitrophenyl-4,5,6,7-tetrahydro-2H-indazole compound of the formula:

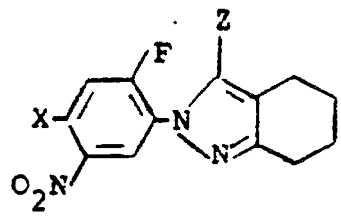

(XI)

wherein X is a chlorine atom or a bromine atom and Z is a chlorine atom or methyl group.

**2.** 2-(4-Chloro-2-fluoro-5-nitrophenyl)-3-methyl-4,5,6,7-tetrahydro-2H-indazole.

**3.** 3-Chloro-2-(4-chloro-2-fluoro-5-nitrophenyl)-4,5,6,7-tetrahydro-2H-indazole.

**4.** A process for the preparation of a compound of as claimed in claim 1 wherein X is a chlorine atom or a bromine atom and Z is a chlorine atom, which process comprises reacting a 2,4-dihalo-5-nitrophenyl-hydrazine of the formula:

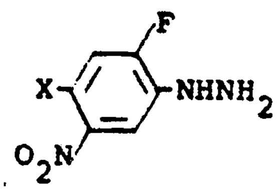

(IX)

wherein X is as defined above with a 2-alkoxycarbonylcyclohexanone of the formula:

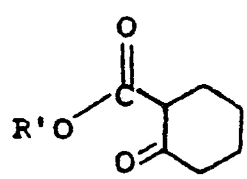

(V)

wherein R' us a $C_1$-$C_4$ alkyl group to give a compound of the formula

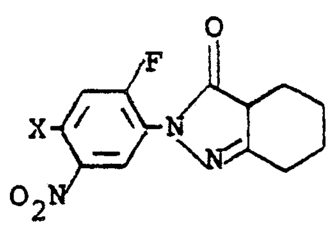

(X)

wherein X is a defined above, followed by reacting the compound of formula (X) with a chlorinating agent.

**5.** A process for the preparation of a compound as claimed in claim 1 wherein X is a chlorine atom or a bromine atom and Z is a methyl group, which process comprises reacting a 2,4-dihalo-5-nitrophenyl-hydrazine of the formula

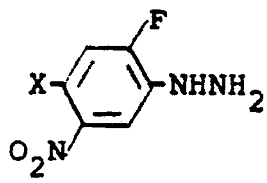

(IX)

wherein X is as defined above with 2-acetyl cyclohexanone.

## Revendications

1. Un 2,4-dihalogéno-5-nitrophényl-4,5,6,7-tétrahydro-2H-indazole, composé de formule :

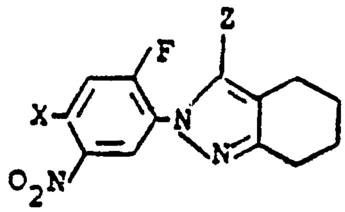

(XI)

dans laquelle x représente un atome de chlore ou un atome de brome, et Z représente un atome de chlore ou un groupe méthyle.

2. Le 2-(4-chloro-2-fluoro-5-nitrophényl)-3-méthyl-4,5,6,7-tétrahydro-2H-indazole.

3. Le 3-chloro-2-(4-chloro-2-fluoro'5-nitrophényl)-4,5,6,7-tétrahydro-2H-indazole.

4. Procédé pour la préparation d'un composé tel que revendiqué à la revendication 1, dans lequel X représente un atome de chlore ou un atome de brome et Z représente un atome de chlore, ce procédé comprenant la réaction d'une 2,4-dihalogéno-5-nitro-phénylhydrazine de formule :

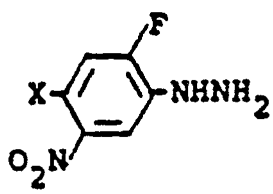

(IX)

(dans laquelle X est tel que défini ci-dessus) avec une 2-alcoxy-carbonylcyclohexanone de formule :

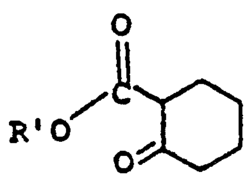

(V)

dans laquelle R' représente un groupe alkyle en $C_1$-$C_4$, ce qui donne un composé de formule:

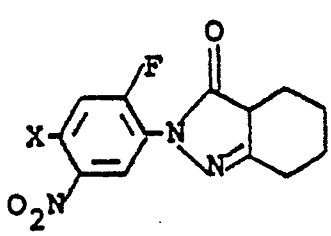

(X)

dans laquelle X est tel que défini ci-dessus, opération suivie de la réaction du composé de formule avec un agent de chloration.

**5.** Procédé pour la préparation d'un composé tel que revendiqué dans la revendication 1, (dans lequel X représente , un atome de chlore ou un atome de brome, et Z représente un groupe méthyle), ce procédé comprenant la réaction d'une 2,4-dihalogéno-5-nitro-phénylhydrazine de formule :

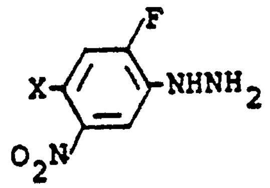

(IX)

(dans laquelle X est tel que défini ci-dessus) avec la 2-acétylcyclo-hexanone.

### Ansprüche

**1.** 2,4-Dihalo-5-nitrophenyl-4,5,6,7-tetrahydro-2H-indazol-Verbindung der Formel

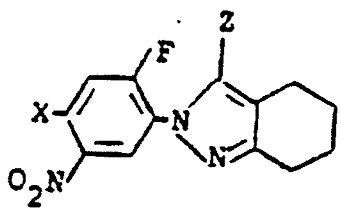

(XI)

in der X ein Chlor- oder Bromatom und Z ein Chloratom oder eine Methylgruppe bedeutet.

**2.** 2-(4-Chlor-2-fluor-5-nitrophenyl)-3-methyl-4,5,6,7-tetrahydro-2H-indazol.

**3.** 3-Chlor-2-(4-chlor-2-fluor-5-nitrophenyl)-4,5,6,7-tetrahydro-2H-indazol.

**4.** Verfahren zur Herstellung einer Verbindung nach Anspruch 1, bei der X ein Chlor- oder Bromatom und Z ein Chlor-atom bedeutet, umfassend die Umsetzung eines 2,4-Dihalo-5-nitrophenylhydrazins der Formel

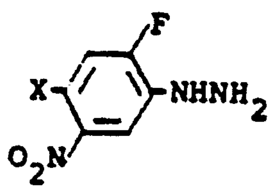

(IX)

in der X wie vorstehend definiert ist, mit einem 2-Alk-oxycarbonylcyclohexanon der Formel

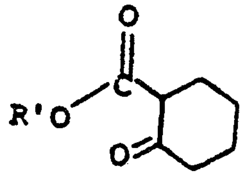

(V)

in der R' einen $C_1$-$C_4$-Alkylrest bedeutet, zu einer Verbindung der Formel

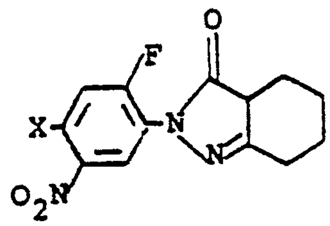

(X)

in der X wie vorstehend definiert ist, und anschließen-des Umsetzen der Verbindung der Formel (X) mit einem Chlorierungsmittel.

5. Verfahren zur Herstellung einer Verbindung nach Anspruch 1, wobei X ein Chlor- oder Bromatom und Z eine Methylgruppe bedeutet, umfassend die Umsetzung eines 2,4-Di-halo-5-nitrophenylhydrazins der Formel

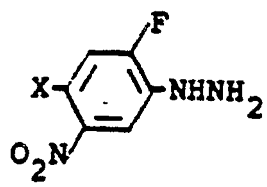

(IX)

in der X wie vorstehend definiert ist mit 2-Acetylcyclo-hexanon.